(19) Europäisches Patentamt
European Patent Office
Office européen des brevets

(11) **EP 0 720 977 B1**

(12) **FASCICULE DE BREVET EUROPEEN**

(45) Date de publication et mention
de la délivrance du brevet:
**13.09.2000 Bulletin 2000/37**

(51) Int Cl.[7]: **C07C 45/72**, C07C 49/17,
B01J 21/10

(21) Numéro de dépôt: **95402631.6**

(22) Date de dépôt: **22.11.1995**

(54) **Aldolisation sélective de l'acétone en diacétone alcool par un catalyseur basique solide**

Selektive Aldolisierung von Aceton in Diaceton Alkohol mit einem festen basischen Katalysator

Selective aldolization of acetone to diacetone alcohol with a solid basic catalyst

(84) Etats contractants désignés:
**BE DE ES FR GB IT NL**

(30) Priorité: **06.01.1995 FR 9500094**

(43) Date de publication de la demande:
**10.07.1996 Bulletin 1996/28**

(60) Demande divisionnaire: **00200485.1 / 1 005 905**

(73) Titulaire: **Atofina**
**92800 Puteaux (FR)**

(72) Inventeurs:
• **Teissier, Rémy**
**F-69340 Francheville (FR)**
• **Tichit, Didier, Le Vallon des Sources-Bât. 1**
**F-34090 Montpellier Cedex (FR)**
• **Figueras, François**
**F-69100 Villeurbanne (FR)**
• **Kervennal, Jacques**
**F-69005 Lyon (FR)**

(56) Documents cités:
EP-A- 0 095 783          DE-C- 800 661
US-A- 4 547 620          US-A- 5 254 743

**Description**

La présente invention se rapporte au domaine technique de la fabrication de diacétonealcool par catalyse basique et plus précisément par catalyse basique hétérogène.

G.S. Salvapati et al, Journal of Molecular Catalysis, 54 (1989), 9-30, ont déjà publié une revue sur les réactions complexes et les nombreux produits que l'on peut obtenir par auto-condensations et trans-condensations des produits cétoniques issus de l'acétone en milieu basique.

L'acétone conduit tout d'abord par une réaction équilibrée au diacétonealcool (DA). Ce dernier peut, soit se déshydrater pour donner une molécule d'oxyde de mésityle (OM), et une molécule d'eau, soit réagir sur une troisième molécule d'acétone pour donner du triacétone-dialcool (TAD).

Craven E.C., J. Appl. Chem (1963) 13, pp. 71-77, avait déjà déterminé que dans la réaction équilibrée la teneur à l'équilibre en DA (en % en poids) dépendait de la température réactionnelle $t_r$ : 0°C (23,1%), 10°C (16,9%), 20°C (12,1%), 30°C (9,1%).

Par ailleurs, on connaît un procédé industriel de fabrication de DA par catalyse à l'aide d'hydroxyde de sodium (2,5 milliéquivalent/kg d'acétone). Ce procédé homogène présente de nombreux inconvénients. Avant la distillation du DA, il faut éliminer la soude par neutralisation à l'acide phosphorique, précipitation du phosphate de sodium et filtration. Or, après ces traitements, dans le mélange contenant le DA, il reste du phosphate de sodium qui va encroûter peu à peu les colonnes de distillation. En conséquence, il faut régulièrement arrêter la distillation pour nettoyer les colonnes, ce qui diminue sensiblement la productivité de l'unité.

Une catalyse hétérogène basique par un catalyseur basique solide facilement séparable de la phase organique permettrait de simplifier le procédé et de supprimer les effluents provenant de la neutralisation de la soude.

Geng Zhang et al, Applied Catalysis, 36 (1988) 189-197 ont étudié l'aldolisation de l'acétone catalysée par des catalyseurs basiques solides tels que Oxyde de magnésium MgO, Oxyde de calcium, CaO, Oxyde de strontium SrO, Oxyde de baryum BaO, Oxyde de lanthane III $La_2O_3$, et Oxyde de zirconium $ZrO_2$.

Ces auteurs ont trouvé que les activités de ces catalyseurs basées sur une même unité de surface spécifique étaient dans l'ordre : BaO> SrO> CaO> MgO> $La_2O_3$> $ZrO_2$. De plus, pour MgO l'addition d'eau et d'ammoniac par pré adsorption entraînait une augmentation marquée de l'activité et de la sélectivité de l'obtention de DA.

Kozo Tanabe et al, Applied Catalysis, 48 (1989) 63-70, ont étudié l'addition des cations métalliques à l'Oxyde de magnésium, pour obtenir un catalyseur d'aldolisation de l'acétone. L'influence des cations $Na^+$, $K^+$, $Rb^+$, $Cs^+$, $Al^{3+}$, $Mn^+$, $Fe^{3+}$, $Co^{2+}$, $Ni^{2+}$, $Cu^{2+}$, $Zn^{2+}$ et $Zr^{4+}$, a été étudié. Ces auteurs ont remarqué que les cations $Na^+$, $ZR4+$ et $Zn^{2+}$ augmentaient efficacement l'activité catalytique pour une teneur en poids de 0,5 à 1 % du cation métallique. L'addition d'eau en des quantités adéquates augmentait à la fois les activités et les sélectivités de ces catalyseurs MgO dopés à l'aide de ces cations. Par contre, ces auteurs ont mis en évidence que l'addition de n'importe quelle concentration de $Al^{3+}$ provoquait une diminution de l'activité, cette diminution indiquée à la figure 3, allait dans le même sens que l'augmentation de la teneur en $Al^{3+}$. Le domaine étudié pour $Al^{3+}$, variant entre 0 % et environ 3 % d'$Al^{3+}$ en poids.

Dans le domaine technique de la chimie minérale, G. Mascolo et O. Marino, Mineralogical Magazine, March 1980, Vol 43. pp. 619-21, ont décrit une nouvelle synthèse de double hydroxydes Mg-Al (DH) ayant une faible teneur en $CO_2$ (0,8-1 %) et de formule générale (I):

$$[Mg^{2+}_{1-x} \ Al^{3+}_x \ (OH)^-_2]^{+x} \qquad [(OH)_x \ (H_2O)_{0,81-x}]^{-x} \qquad (I)$$

avec $0,23 \leq x \leq 0,33$

Ces DH ont une structure cristalline similaire à celles de l'hydrotalcite ou de la manasseite naturelle avec un réseau cristallin rhomboédrique.

Si $x_{Mg}$ est défini par le rapport molaire:

$x_{Mg}$ = Mg/(Mg + Al), le domaine de composition du DH pur existe pour les valeurs de $x_{Mg}$ telles que $0,67 \leq x_{Mg} \leq 0,77$.

Les paramètres de la maille cristalline a et c sont pour les compositions limites :

a = 3,038 Å

c = 22,6 Å, pour la composition limite riche en $Al^{3+}$, et

a = 3,054

c = 23,4 Å, pour la composition limite riche en $Mg^{2+}$.

Ces DH se composeraient donc, comme l'hydrotalcite naturelle, de couches du type brucite chargées positivement de formule $[Mg_{1-x} \ Al_x \ (OH)_2]$ et d'inter-couches constituées d'hydroxyles OH- et de molécules d'eau.

Dans la famille des DH, on connaît un minerai naturel, la Meixnerite, de formule $Mg_6Al_2(OH)_{18}. \ 4H_2O$ contenant moins de 2 % de $CO_2$, mais qui absorbe avidement le $CO_2$ de l'air pendant tout broyage du minerai à l'air.

La formule de la Meixnerite exprimée comme une sorte de cas particulier de la formule générale (I) est :

$$[Mg^{2+}_{1-0,25} \; Al^{3+}_{0,25} (OH)^-_2]^{+0,25} \quad [(OH)_{0,25} (H_2O)_{0,5}]^{-0,25},$$

soit x = 0,25.

Les DH de G. Mascolo et O. Marino sont préparés à partir de MgO obtenu par calcination de carbonate basique de magnésium à 650°C pendant 6 heures et de gel d'alumine Merck. Ces deux composants sont introduits dans un rapport molaire déterminé $X_m$ et mis en suspension dans de l'eau distillée, dans un récipient fermé en Téflon, sous agitation, pendant une semaine à 80± 1°C. La suspension est ensuite filtrée à l'abri du $CO_2$ et enfin le solide recueilli séché sur silicagel.

Selon Allmann, Fortschr. Minéral 1971, 48, 24-30, dans les composés du type hydrotalcite contenant des anions et des molécules d'eau entre les couches chargées du type brucite de formule:

$$[Mg_{1-x}Al_x(OH)_2],$$

x a une valeur telle que $0,20 \leq x \leq 0,33$.

EP-A-0 095 783 concerne l'aldolisation de l'acetone en l'oxyde mesitylene et isophorone, par utilisation comme catalyseur d'une argile anionique synthétique traîtée à haute temperature.

Il a été trouvé par la présente invention, que les DH de G. Mascolo et O. Marino constituaient d'excellents catalyseurs d'aldolisation sélective de l'acétone en DA. De plus il a été mis au point un nouveau procédé de synthèse des composés de formule générale (II) dans laquelle x a une valeur telle que $0,20 \leq x \leq 0,33$.

Plus précisément, la présente invention réside tout d'abord en un procédé d'aldolisation sélective d'acétone en diacétonealcool (DA) en présence d'un catalyseur basique solide, caractérisé par le fait que le catalyseur a la formule générale (II):

$$[Mg^{2+}_{1-x} \; Al^{3+}_x (OH)^-_2]^{+x} \quad [(OH)^-_x (H_2O)_n]^{-x} \qquad (II)$$

avec $0,20 \leq x \leq 0,33$,

n ayant une valeur inférieure à 1, ce catalyseur ayant une structure cristalline définie, analogue à celle de l'hydrotalcite ou encore de la meixnerite.

De préférence, n a une valeur telle que
$0,5 \leq n \leq 0,75$.

Avantageusement, n à une valeur égale ou voisine de 0,81 -x et inclus la valeur n = 0,5 de la Meixnerite pour laquelle x vaut 0,25.

La présente invention propose également un procédé dans lequel on réhydrate, à l'abri de $CO_2$, un oxyde mixte de magnésium et d'aluminium, et plus précisément un procédé de préparation du catalyseur de formule générale (II), caractérisé par le fait qu'il comporte les étapes suivantes:

a) on calcine une hydrotalcite dans laquelle
x a une valeur telle que $0,20 \leq x \leq 0,33$, à une température inférieure à 800°C, pour obtenir un oxyde mixte de magnésium et d'aluminium,
b) l'oxyde mixte ainsi obtenu est réhydraté par de l'eau en l'absence de $CO_2$.

Les catalyseurs (II), très basiques, ont une tendance naturelle à se carbonater en présence d'air. Leur préparation et leur utilisation doivent avoir lieu dans un milieu exempt de gaz carbonique. Le remplacement partiel de l'anion (OH)$^-$ par l'anion carbonate $CO_3^{2-}$ diminue seulement partiellement les propriétés catalytiques du catalyseur (I). Par contre, l'hydrotalcite de formule :

$$[Mg^{2+}_{1-0,25} \quad Al^{3+}_{0,25} (OH)^{-}_{2}]^{+0,25} \quad [(CO_3)^{2-}_{0,125} (H_2O)_{0,5}]^{-0,25}$$

ne possède plus de propriété catalytique notable dans l'aldolisation de l'acétone en DA.

La présente invention sera encore mieux comprise grâce à la partie expérimentale suivante :

## Partie Expérimentale

1) Préparation des catalyseurs de formule générale (II)

En plus de la préparation de Mascolo et Marino rapportée ci-dessus, deux procédés de synthèse ont été utilisés :

- En partant d'une hydrotalcite synthétisée selon S. Myata, Clays and Clay Minerals, (1980) 28, pp 50-56.
- En partant d'un double oxyde de magnésium et d'aluminium commercialisé par la Société Japonaise Kyowa sous la référence KW 2000.

1.1) Produit de départ : hydrotalcite

1.1.1) Synthèse de l'hydrotalcite

Cette synthèse est effectuée selon la méthode de S. Myata par mélange en milieu alcalin d'une solution de $MgCl_2$ avec une solution de $AlCl_3$.

On obtient après filtration et séchage une hydrotalcite de rapport molaire Mg/Al de 2,27 (soit x = 0,306). La surface spécifique mesurée par la méthode BET est 80 $m^2/g$.

1.1.2) Echange au carbonate

Le produit obtenu ci-dessus est échangé deux fois comme suit : à une solution de 0,69 g de $Na_2CO_3$ dans 100 ml d'eau distillée, on ajoute deux grammes de l'hydrotalcite. Sous agitation, on porte la suspension à 80°C durant trois heures. On filtre, puis on lave deux fois à l'eau. On effectue un second échange de la même manière. On obtient un solide où aucun chlorure n'est décelable par potentiométrie.

1.1.3) Calcination

Le solide échangé ci-dessus est calciné sous air sec selon le programme thermique suivant :

- montée à 450°C en 1 heure ou plus,
- palier à 450°C pendant 10 heures,
- refroidissement sous balayage à l'air sec exempt de $CO_2$.

Le poids du solide calciné représente 60 à 65 % du poids de solide initial.

1.1.4) Activation

A température ambiante, l'air sec est remplacé par un gaz exempt de $CO_2$, tel que de l'azote et qui a traversé auparavant un saturateur rempli d'eau. Pour un poids initial de 5 g d'hydrotalcite, on envoie un débit de 5,5 litres d'azote humide pendant 16 h, soit un volume total de 18 l d'azote humide par gramme. Le solide obtenu de formule générale II dans laquelle x vaut 0,306, est immédiatement testé dans le test catalytique qui sera décrit plus loin.

1.2.) Produit de départ : KW 2000

Cet oxyde double, a les caractéristiques suivantes:

Formule chimique :

$$4,5 \text{ MgO. Al}_2\text{O}_3 \text{ (x = 0,3077)}$$

| | |
|---|---|
| Masse volumique apparente : | 44 ml/10g |
| Aspects : | poudre blanche, fine, sans odeur BET (m$^2$/g) : 172 |
| Taille moyenne de particule : | 70 µm |
| Propriété déshydratante : | absorbe au maximum 70-80 parties d'eau pour 100 parties de KW 2000. |

1.2.1) Hydratation par de l'eau en phase liquide

Six grammes de KW 2000 sont ajoutés sous agitation à 200 ml d'eau décarbonatée (eau permutée, puis bouillie). On laisse trois heures, puis on évapore sous vide l'eau vers 40°C. On obtient 9g de solide qui est conservé à l'abri de $CO_2$ pendant 1 nuit, avant d'être broyé rapidement toujours à l'abri d'un contact avec du $CO_2$. On obtient ainsi un solide divisé de formule générale (II) où x vaut 0,3077 et qui a une structure cristalline du type de l'hydrotalcite ou de la Meixnerite.

1.2.2) Hydratation par de l'eau en phase vapeur.

Dix grammes de KW 2000 sont disposés en couche fine dans un cristallisoir. Le cristallisoir est introduit dans un dessiccateur au fond duquel on a versé environ 20 g d'eau, après que le dessiccateur ait été préalablement purgé à l'azote pour éliminer toute trace en $CO_2$.

On laisse durant 3 jours au moins le solide dans cette atmosphère saturée en eau.

Après cinq jours, le solide pèse 13,3g.

On obtient ainsi un solide sous forme de poudre de formule générale (II) où x vaut 0,3077 et qui a la même structure cristalline que le solide obtenu en 1.2.1).

2) Test catalytique

Dans un réacteur de 500 ml agité, muni d'un condenseur à reflux, d'un système d'inertage à l'azote et d'une double enveloppe permettant de le thermostater par circulation d'une fluide thermique, on introduit 100 g d'acétone commerciale Aldrich (ref 17,912-4).

Sous agitation, on porte l'acétone à 0°C en purgeant le ciel du réacteur à l'azote.

Lorsque la température est stabilisée, on introduit la charge catalytique en évitant au maximum tout contact de celle-ci avec l'air.

On laisse réagir sous agitation. On prélève à intervalles réguliers un échantillon que l'on analyse en chromatographie phase gazeuse (CPG).

Comme le milieu réactionnel est composé d'un liquide organique et du catalyseur basique solide, il est très facile de séparer ces deux phases, par exemple par décantation ou filtration à l'abri du $CO_2$, donc de l'air.

On suit ainsi la formation du Diacétonealcool (DA), de l'Oxyde de mésityle (OM) et du Triacétone-dialcool (TAD). Les caractéristiques de l'analyse CPG sont les suivantes :

- colonne capillaire ayant une longueur de 25 m, un diamètre de 0,55 mm, Hewlett-Packard HP5-(Si8)
- Détection par ionisation de flamme (FID),
- Gaz vecteur : $N_2$ 6 ml/min.,
- Température de colonne : 4 min. à 60°C, puis 6°C/min. jusqu'à 180°C,
- Injecteur : température 150°C
- Détecteur : température 200°C,

  Etalon interne : xylène

2.1) Exemple 1

Test à l'aide du catalyseur 1.1.4 (x = 0,306)

Cet exemple figure dans le tableau I

TABLEAU I

| DUREE EN HEURE | DA % EN POIDS | TAD % EN POIDS | OM % EN POIDS |
|---|---|---|---|
| 0 | 0 | 0 | 0 |
| 1 | 16,5 | 0,6 | 0 |
| 2 | 21 | 0,8 | 0 |
| 3 | 22,9 | 0,85 | 0 |
| 4 | 24,6 | 0,9 | 0 |

2.2) Exemple comparatif 2

A titre de comparaison, avec la soude (NaOH) à la concentration de 2,5 m.éq/kg d'acétone, en substitution au catalyseur hétérogène de l'exemple 1, on obtient dans les mêmes conditions expérimentales les résultats suivants visibles au tableau II

TABLEAU II

| DUREE EN HEURE | DA % EN POIDS |
|---|---|
| 0 | 0 |
| 1 | 13 |
| 2 | 14,5 |
| 3 | 18 |
| 5 | 18,5 |

2.3.) Exemple comparatif 3

A titre de comparaisons, le solide calciné 1.1.3 est utilisé dans le test catalytique. Les résultats figurent dans le Tableau III.

TABLEAU III

| DUREE EN HEURE | DA % EN POIDS |
|---|---|
| 0 | 0 |
| 1 | 0,8 |
| 2 | 1,4 |
| 3 | 2 |
| 5 | 2,9 |
| 24 | 7 |

2.4) Exemple comparatif 4

Au lieu de partir d'hydrotalcite selon la préparation 1.1.), nous avons utilisé du MgO. Ce MgO a été calciné suivant 1.1.3) puis activé selon 1.1.4). Ce type de catalyseur correspond à ceux décrits par Geng Zhang et al (voir ci-dessus). Le test est réalisé dans les mêmes conditions générales. Les résultats figurent dans le tableau IV.

TABLEAU IV

| DUREE EN HEURE | DA % EN POIDS | TAD % EN POIDS |
|---|---|---|
| 1 | 4,5 | - |
| 2 | 6,8 | 0,2 |

TABLEAU IV   (suite)

| DUREE EN HEURE | DA % EN POIDS | TAD % EN POIDS |
|---|---|---|
| 3 | 8,5 | 0,25 |
| 8 | 13 | - |
| 24 | 19 | 0,6 |

2.5- 2.6- 2.7 Exemples 5,6 et 7

Nous avons fait varier la quantité d'azote humide de l'activation 1.1.4) de l'hydrotalcite calcinée. Ces activations sont conduites à la même température de 20°C. On fait varier le débit de gaz, donc la quantité d'azote humide utilisée par gramme de solide calciné. Les catalyseurs obtenus sont définis ci-après par un coefficient d'activité $C_a$, ce dernier étant égal à la masse de DA formée au cours de la première heure du test, divisée par la masse du catalyseur mis en oeuvre. Les résultats apparaissent dans le tableau V.

TABLEAU V

| EXEMPLE | $N_2$ HUMIDE EN LITRE PAR G DE CATALYSEUR | $C_a$ |
|---|---|---|
| 5 | 5 | 0,2 |
| 6 | 11 | 1,5 |
| 7 | 25 | 3,4 |

2.8) Exemple 8

Le catalyseur solide obtenu en 1.2.1) par hydratation par de l'eau en phase liquide de KW 2000 est testé de la manière suivante:

4,5 g de ce solide broyé sont introduits dans 100 g d'acétone préalablement refroidi à 0° C et sous atmosphère d'azote. On laisse réagir sous agitation. Au bout d'une heure de réaction, on obtient 13,75 g de DA, soit un coefficient $c_a$ égal à 3.

2.9) Exemple 9

Le catalyseur solide obtenu en 1.2.2) par hydratation par de l'eau en phase vapeur de KW 2000 est testé de la même manière que dans l'exemple 8 précédent, mais avec une quantité de solide de 2,9 g.

Le coefficient d'activité $c_a$ est égal à 4,1.

2.10) Exemple 10

Le catalyseur de l'exemple 1 a été recueilli par filtration rapide, puis recyclé 7 fois dans le test défini ci-dessus.

Les résultats figurent dans le tableau VI

TABLEAU VI

| NOMBRE DE RECYCLAGES | $C_a$ |
|---|---|
| 1 | 3,6 |
| 2 | 3,7 |
| 3 | 3,3 |
| 4 | 3,3 |
| 5 | - |
| 6 | 3,6 |
| 7 | 3,7 |

Ces 7 recyclages n'entraînent pas de perte d'activité du catalyseur.

2.11) Exemple 11

Le catalyseur de l'exemple 6 a été recueilli par filtration rapide, puis séché en étuve ventilée à 100°C pendant une nuit. Ensuite il a été soumis à la calcination selon 1.1.3) puis activé selon 1.1.4) et enfin soumis au test. Son coefficient d'activité $C_a$ est de 3.8.

En conclusion, les exemples ci-dessus montrent que les catalyseurs selon l'invention permettent une très bonne conversion en DA. Le taux de conversion arrive à égaler la valeur d'équilibre thermodynamique à 0° C, avec une excellente sélectivité ( au moins 97% ). De plus, ces catalyseurs conservent leur activité dans le recyclage.

**Revendications**

1.   Utilisation d'un catalyseur basique solide de formule générale (II):

$$[(Mg^{2+})_{1-x}(Al^{3+})_x(OH^-)_2]^{+x}[(OH)_x(H_2O)_n]^{-x} \tag{II}$$

avec $0,20 \leq x \leq 0,33$, n ayant une valeur inférieure à 1, et ayant une structure cristalline définie, analogue à celle de l'hydrotalcite ou encore de la Meixnerite pour l'aldolisation sélective d'acétone en diacétonealcool (DA).

2.   Utilisation suivant la revendication 2, caractérisée par le fait que n vérifie la relation $0,5 \leq n \leq 0,75$.

3.   Utilisation suivant la revendication 2, caractérisée par le fait que n a une valeur égale ou voisine de 0,81-x et inclus la valeur n = 0,5 de la Meixnerite pour laquelle x vaut 0,25.

**Patentansprüche**

1.   Verwendung eines festen basischen Katalysators der allgemeinen Formel (II):

$$[(Mg^{2+})_{1-x}(Al^{3+})_x(OH^-)_2]^{+x}[(OH)_x(H_2O)_n]^{-x} \tag{II}$$

mit $0,20 \leq x \leq 0,33$, wobei n einen Wert kleiner als 1 hat und eine bestimmte Kristallstruktur aufweist, die analog zu der des Hydrotalcits oder auch des Meixnerits ist, für die selektive Aldolkondensation von Aceton zu Diacetonalkohol (DA).

2.   Verwendung nach Anspruch 1, dadurch gekennzeichnet, daß n der Beziehung $0,5 \leq n \leq 0,75$ entspricht.

3.   Verwendung nach Anspruch 2, dadurch gekennzeichnet, daß n einen Wert gleich oder nahe 0,81-x hat und den Wert n = 0,5 des Meixnerits, für den x 0,25 ist, einschließt.

**Claims**

1.   Use of a solid basic catalyst of general formula (II) :

$$[(Mg^{2+})_{1-x}(Al^{3+})_x(OH^-)_2]^{+x}[(OH^-)_x(H_2O)_n]^{-x} \tag{II}$$

with $0.20 \leq x \leq 0.33$, and n having a value of less than 1 having a defined crystal structure similar to that of hydrotalcite or, alternatively, of meixnerite, for the selective aldolization of acetone to diacetone alcohol (DA).

2.   Use according to Claim 1, characterized in that n verifies the relationship $0.5 \leq n \leq 0.75$.

3.   Use according to Claim 2, characterized in that n has a value equal to or in the region of 0.81-x and includes the value n = 0.5 of meixnerite for which x has the value 0.25.